# EUROPÄISCHE PATENTSCHRIFT

(11) **EP 2 315 825 B1**
(45) Veröffentlichungstag und Bekanntmachung des Hinweises auf die Patenterteilung: **25.01.2012**
(21) Anmeldenummer: 09777691.8
(22) Anmeldetag: 06.08.2009
(51) Int. Cl.: C12N 1/06

(54) **ZELLAUFSCHLUSS PFLANZLICHER ODER TIERISCHER AUSGANGSMATERIALIEN MITTELS KOMBINATION VON SPRÜHVERFAHREN UND DEKOMPRESSION ZUR SELEKTIVEN EXTRAKTION UND ABSCHEIDUNG INTRAZELLULÄRER WERTSTOFFE**
CELL LYSIS OF PLANT OR ANIMAL STARTING MATERIALS BY A COMBINATION OF A SPRAY METHOD AND DECOMPRESSION FOR THE SELECTIVE EXTRACTION AND SEPARATION OF VALUABLE INTRACELLULAR MATERIALS
RUPTURE CELLULAIRE DE MATIÈRES DE DÉPART VÉGÉTALES OU ANIMALES, PAR COMBINAISON D'UN PROCÉDÉ PAR PULVÉRISATION ET D'UNE DÉCOMPRESSION, POUR EXTRACTION SÉLECTIVE ET SÉPARATION DE MATIÈRES RÉUTILISABLES INTRACELLULAIRES

(30) Priorität: 07.08.2008 DE 102008036723
(43) Veröffentlichungstag der Anmeldung: 04.05.2011
(73) Patentinhaber: Uhde High Pressure Technologies GmbH, 58093 Hagen (DE)
(72) Erfinder: DIERKES, Heribert, 58093 Hagen (DE); STEINHAGEN, Volkmar, 58097 Hagen (DE); BORK, Michael, 44329 Dortmund (DE); LÜTGE, Christoph, 59425 Unna (DE); KNEZ, Zeljko, 2000 Maribor (SI)
(86) Internationale Anmeldenummer: PCT/EP2009/005689
(87) Internationale Veröffentlichungsnummer: WO 2010/015398

(56) Entgegenhaltungen:
- WO-A-91/01367
- US-A- 5 306 637
- US-A- 5 380 826

## Beschreibung

Die Erfindung betrifft ein Verfahren zum Zellaufschluss aus pflanzlichen oder tierischen Ausgangsmaterialen mittels Sprühverfahren kombiniert mit der Dekompression der Zellen zur anschließenden selektiven Extraktion und Abscheidung enthaltener Inhaltsstoffe.

In diesem Zusammenhang bekannte mechanische Zellaufschlussmethoden sind zum Einen die Homogenisierung mit rotierenden Messern, die meist unter hohem Druck durchgeführt wird, der Aufschluss in einer Rührwerkskugelmühle, das Pressen einer Probe unter hohem Druck durch eine schmale Öffnung oder auch die Methode mittels Ultraschallhomogenisator.

Nachteil der genannten mechanischen Methoden sind die auf die Zellen wirkenden Scherkräfte aufgrund von Reibung, deren Überwindung bei hohen Geschwindigkeiten zur Wärmebildung führt und in der Folge eine hohe Temperatur erzeugt. Dies kann einen schädigenden Einfluss auf die Inhaltsstoffe der entstehenden Zellextrakte haben.

Eine schonendere Methode stellt die physikalische Dekompression der Zellen dar. Hierbei wird entsprechend dem Henry'schen Gesetz Suspensionsgas in Zellen bei höheren Gas-Drücken angereichert und die Zellmembranen werden durch eine schlagartige Druckentlastung zum Platzen gebracht. Dies geschieht dadurch, dass das gelöste Gas nicht schnell genug entweichen kann und innerhalb der Zellen in Form größer werdender Gasbläschen ausperlt. Dadurch steigt die mechanische Belastung, die auf die Zelle wirkt, solange an, bis die Zelle platzt und der Zellinhalt freigesetzt wird.

Nachteil der Dekompressionsmethode ist vor allem, dass nur relativ leicht aufzubrechende Zellen effektiv aufgeschlossen werden können, was eine zusätzliche Anwendung von nicht-mechanischen Aufschlussverfahren, wie den Einsatz von Enzymen, erforderlich macht. Dies wiederum macht ein Aufschlussverfahren im Großmaßstab unrentabel.

Nach dem Stand der Technik erfolgt die Extraktion mittels überkritischen Fluiden. Darunter werden Gase oder Flüssigkeiten verstanden, die sich oberhalb ihrer kritischen Temperatur und ihres kritischen Drucks befinden, die im jeweiligen Phasendiagramm der reinen Substanz definiert sind. Der Vorteil liegt in einer erhöhten Löslichkeit für schwer lösliche Stoffe im überkritischen Bereich. Darüber hinaus kann die Löslichkeit noch über Änderungen von Druck oder Temperatur kontrolliert werden. Ein Beispiel ist die Entkoffeinierung einer Teepflanze mittels überkritischem CO₂, wie in WO 2008/05537 A1 beschrieben. Aber auch weitere Anwendungen in der Chemischen Industrie und der Lebensmittelindustrie haben sich inzwischen etabliert, wie z. B. die Extraktion von Ölen, Ingwer, schwarzem Pfeffer oder Chilipulver mittels überkritischem CO₂ oder überkritischem Propan.

Die Veröffentlichung WO 2008/061716 A1 beschreibt noch eine weitere Verbesserung der Methode der Extraktion, bei der auf Schleppmittel verzichtet wird, die normalerweise dazu eingesetzt werden, um eine weitere Erhöhung der Löslichkeit zu gewährleisten, ohne den Druck weiter zu erhöhen.

In EP 0 941 140 B1 wird die Extraktion von verschiedenen Produkten aus einem Fermentationsmedium, mittels Kohlenstoffdioxid, das sich im überkritischen oder nahe des kritischen Zustandes befindet, beschrieben. Die Extraktion, die in diesem Patent beschrieben wird, geht von einer Suspension auf Wasserbasis aus. Es findet sich keinerlei Hinweis auf einen simultanen Zellaufschluss und der Extraktion der Substanzen. Da das überkritische bzw. das sich nahe dem kritischen Zustand befindliche Lösungsmittel Kohlenstoffdioxid war, was eine relativ gute Löslichkeit in Wasser aufweist, ist zu erwarten, dass Extrakte mit einem hohen Wassergehalt erhalten werden. Die gewählten Extraktiontemperaturen sind zudem relativ hoch, wodurch verschiedenste hydrolytische Reaktionen der extrahierten Substanzen mit Wasser erfolgen können.

Die US 5,306,637 beschreibt eine Methode zum Zellaufschluss, bei der ein enzymatischer Aufschluss gekoppelt wird mit einer nachfolgenden plötzlichen Entspannung, wodurch die Zellen platzen und der in ihnen enthaltene Wertstoff effektiv freigesetzt wird. Allerdings sind die eingesetzten Drücke gering, so dass mit einer langen Sättigungszeit zu rechnen ist. Diese Erfindung nutzt Kohlenstoffdioxid , das sich im überkritschen oder nahe des überkritischen Zustandes befindet, dem optional Schleppmittel wie Schwefeloxid, Stickstoffmonoxid, Wasserstoffperoxid, Ethanol oder Mischungen dieser Schleppmittel zugesetzt werden, für den Aufschluß mikrobieller Zellen sowie für die Extraktion intrazellulärer Komponenten, wie Proteinen oder Nukleinsäuren. Die Isolierung von Proteinen oder Nukleinsäuren basiert nicht auf die Löslichkeit dieser Substanzen in den verwendeten Gasen, sondern beruht auf der Präzipitation dieser Substanzen aus der Suspension. Die Rückgewinnung der Gasmischungen ist dabei äußerst problematisch und der hier beschriebene Prozess findet deshalb im Industriemaßstab keine Anwendung.

In der WO91/01367 wird zunächst ein Lösungsmittel, das als Gas vorliegt und das eine kritische Temperatur aufweist, die zwischen 0 und 100°C liegt, ausgewählt. Dieses Lösungsmittel wird auf einen Druck, der in der Nähe des kritischen Drucks des jeweiligen Lösungsmittels oder aber höher liegt und auf eine Temperatur, die in der Nähe der kritischen Temperatur des jeweiligen Lösungsmittels liegt, gebracht. Das Lösungsmittel wird anschließend mit einer Suspension des Zellmaterials zusammengebracht, um die Zellen mit dem Lösungsmittel unter den aufgeführten Bedingungen zu sättigen. Im nächsten Schritt wird der Druck abgesenkt, was in einer teilweisen Zerstörung der Zellmembran und dem Freisetzen von zellulären Komponenten führt. Daraufhin wird das aufgeschlossene Zellmaterial in einen zweiten Kessel eingebracht. Wie aus dem Stand der Technik aber bekannt ist, ist damit zu rechnen, dass die freigesetzten Proteine und Nukleinsäuren, die auf diese Weise in Lösung gebracht werden sollen, in Lösungsmitteln, die sich in überkritischen Zustand oder nahe des kritischen Zustands befinden, eine äußert geringe Löslichkeit aufweisen.

Daraus folgt, dass trotz der permanenten Verbesserung, der im Stand der Technik zu findenden Methoden zum Zellaufschluss immer noch erhebliche Rückstände der zu isolierenden Substanz im Zellextrakt verbleiben.

Somit besteht nach wie vor das Bedürfnis, durch Verbesserungen der bestehenden Prozesse eine größtmögliche Ausbeute und Reinheit der gewünschten, schwer löslichen intrazellulären Wertstoffe aus dem Zellextrakt in einem superkritischen Lösungsmittel oder einem Lösungsmittel, das sich nahe des kritischen Zustandes befindet, zu erzielen, was sich die hier zu beschreibende Erfindung zur Aufgabe macht.

Diese Aufgabe wird gelöst mittels dem erfindungsgemäßen Verfahren zum Zellaufschluss von biogenen, suspendierten Ausgangsmaterialen mittels Kombination von Druckbeaufschlagung, Versprühen und Dekompression mit anschießender selektiver Extraktion und Abscheidung zellulärer Wertstoffe, wobei mindestens ein Vorratsbehälter als Vorlage für eine Suspension aus biogenem Ausgangsmaterial dient und mindestens ein weiterer Vorratsbehälter als Vorlage für ein Lösungsmittel verwendet wird, in einer Einheit zum Zellaufschluss ein Zellextrakt hergestellt wird, nachfolgend in einer Extraktionsstufe das Zellextrakt von Gas durchströmt wird und das mit zellulären Wertstoffen beladene Gas in einer Abscheidestufe unter Druckabsenkung von den zellulären Wertstoffen abgetrennt wird. Die Suspension aus biogenem Ausgangsmaterial wird dabei mittels einer Vorrichtung zur Druckerhöhung auf einen Druck von 100 - 2500 bar gebracht, das Lösungsmittel wird ebenfalls mittels einer Vorrichtung zur Druckerhöhung auf einen Druck von 100 - 2500 bar gebracht, daraufhin wird das Lösungsmittel und die Suspension in einer Leitung unter einem Druck von 100 - 2500 bar zusammengeführt und zu einem Lösungsgemisch vermischt, und anschließend wird das Lösungsgemisch über mindestens eine Düse unter einem Druck von 100 - 2500 bar und einer Temperatur von 10 - 90°C in einen Behälter, der einen geringeren Druck aufweist, versprüht. Mittels dieser Methode wird erreicht, dass der Zellaufschluss des biogenen, suspendierten Ausgangsmaterials und die Lösung der zellulären Wertstoffe simultan erfolgen.

Dabei gilt im Allgemeinen, dass sich der Zellaufschluss verbessert, je höher der Druck des Lösungsgemisches aus biogenem, suspendiertem Zellmaterial und Lösungsmittel gewählt wird. In Hinblick auf Equipmentkosten und aus prozesstechnischen Gründen ist es dabei am vorteilhaftesten, sich in einem Druckbereich zwischen 1300 und 1600 bar zu bewegen.

In einer Ausgestaltung des Verfahrens wird das Lösungsmittel, mit dem die Suspension aus biogenem Ausgangsmaterial gemischt wird, aus einer Gruppe ausgewählt, die Ethan, Ethylen, Propan, Propylen, Butan, Buthylen, weitere gesättigte oder ungesättigte Kohlenwasserstoffe, Kohlenstoffdioxid, Lachgas, Dimethylether, Schwefelhexaflourid, R 134a, R125, R32, R141b, und Mischungen daraus, enthält. Optional ist Freone, wie zum Beispiel das Lösungsmittel, mit dem die Suspension aus biogenem Ausgangsmaterial gemischt wird, ein überkritisches Fluid, welches kein Kohlenwasserstoff ist. Das Lösungsmittel, das sich im überkritischen oder nahe dem kritischen Zustand befindet, zeichnet sich dadurch aus, dass Wasser darin praktisch nicht löslich ist.

Optional kann die Suspension aus biogenem Ausgangsmaterial vor dem Versprühen mit dem Lösungsmittel gesättigt oder übersättigt werden.

In einer weiteren vorteilhaften Ausgestaltung des Verfahrens wird das biogene Ausgangsmaterial vor Bildung der Suspension durch Waschen, Filtrieren, Zerkleinern, Mahlen oder Sieben vorbehandelt.

Weitere Ausgestaltungsmöglichkeiten des erfindungsgemäßen Verfahrens zum Zellaufschluss von biogenem, suspendiertem Ausgangsmaterial mittels Druckbeaufschlagung, Versprühen und Dekompression beziehen sich auf die Verknüpfung mit anderen Aufschlussverfahren. Dabei werden diese Aufschlussverfahren entweder aus einer Gruppe mechanischer Verfahren ausgewählt, die den Zellaufschluss mittels Hochdruckhomogenisator, Kugelmühle, Ultaschallhomogenisator, French Press und Prallstrahlapparaturen enthält. Alternativ werden diese Aufschlussverfahren aus einer Gruppe chemischer Methoden ausgewählt, die den Zellaufschluss mittels Antibiotika, Chelatbildner, Chaotrope Agenzien, Detergenzien und Alkalische Behandlung enthält. Dabei werden die Zellwände entweder permeabilisiert oder verseift. Desweiteren besteht die Möglichkeit, das erfindungsgemäße Verfahren mit einem Aufschlussverfahren zu verknüpfen, das aus einer Gruppe biologischer Methoden ausgewählt wird, die den Zellaufschluss mittels Enzymen, Phagen oder Autolyse enthält. Optional kann dieses Verfahren auch aus einer Gruppe physikalischer Methoden ausgewählt werden, die den Zellaufschluss mittels Einfrieren und Auftauen, Thermolyse oder Dekompression enthält.

Optional besteht das verwendete biogene, suspendierte Ausgangsmaterial aus Algen.

Weitere Ausgestaltungsmöglichkeiten des Verfahrens beziehen sich auf die zu extrahierenden zellulären Wertstoffe. Dies sind zum Einen zelluläre Wertstoffe der Carotenoidklasse, wie Carotine oder Xanthophylle. Alternativ handelt es sich um den zellulären Wertstoff Astaxanthin, der extrahiert werden soll. Desweiteren kommt die Exraktion zellulärer Wertstoffe aus der Stoffklasse der Fette und Öle in Betracht, die im biogenen suspendierten Ausgangsmaterial enthalten sind.

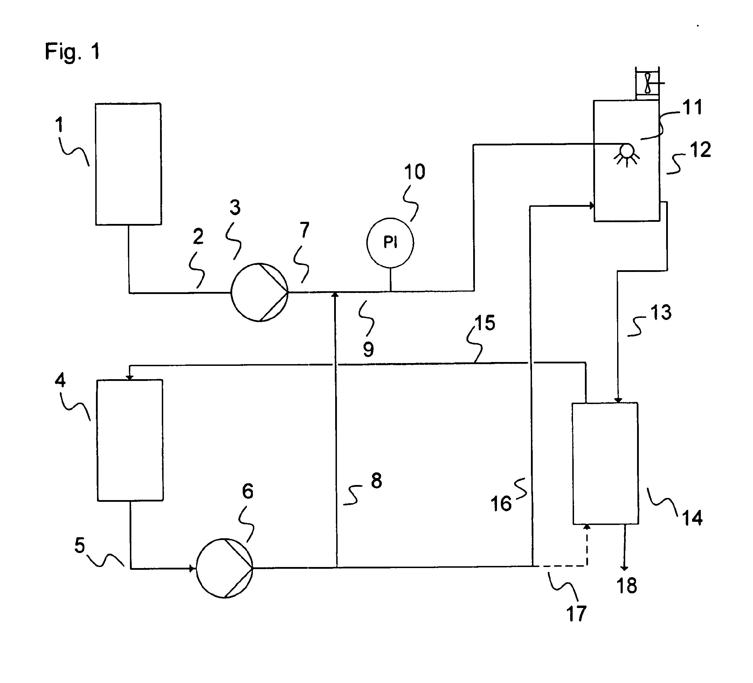

Ein Ausführungsbeispiel der Erfindung ist in Fig. 1 dargestellt und wird im Folgenden näher beschrieben. Es zeigt:
- Fig. 1:: Eine erfindungsgemäße Verfahrensskizze des Verfahrens zum Zellaufschluss von biogenen, suspendierten Ausgangsmaterialen mittels Kombination von Druckbeaufschlagung, Versprühen und Dekompression mit anschließender selektiver Extraktion und Abscheidung zellulärer Wertstoffe.

Fig. 1 zeigt einen Vorratsbehälter **1** zur Aufnahme des biogenen, suspendierten Ausgangsmaterials, sowie einen weiteren Vorratsbehälter **4** zur Aufnahme des Lösungsmittels. Das biogene, suspendierte Ausgangsmaterial **2** wird über eine Pumpe **3** auf einen Druck zwischen 100 und 2500 bar gebracht. Das Lösungsmittel **5** wird über eine Pumpe 6 ebenfalls auf einen Druck zwischen 100 und 2500 bar gebracht. Dabei kann das biogene, suspendierte Ausgangsmaterial **2** und das Lösungsmittel **5** auf den gleichen Druck gebracht werden oder alternativ können sich die Drücke unterscheiden. Nachfolgend wird das unter Druck stehende biogene Ausgangsmaterial **7** dann mit dem unter Druck stehenden Lösungsmittel **8** in einer Leitung zu einem Lösungsgemisch **9** zusammengeführt, die ebenfalls unter einem Druck zwischen 100 und 2500 bar steht. Dieser Leitungsdruck wird über ein Druckmessgerät **10** kontrolliert. Das Lösungsgemisch **9** wird daraufhin in einen Behälter **12** eingebracht, der einen geringeren Druck als das Lösungsgemisch aufweist, wobei die Einbringung des Lösungsgemisches über Düsen **11** erfolgt, die das Lösungsgemisch **9** in den Behälter **12** einsprühen. Damit erfolgen also die Druckabsenkung und der Versprühvorgang des Lösungsgemisches **9** simultan. Dem Behälter **12** wird ein Lösungsmittel zur Extraktion **16** zugeleitet, das im Gegenstrom zum Lösungsgemisch **9** zugeführt wird. Das Lösungsmittel zur Extraktion **16** stammt dabei aus dem Vorratsbehälter **4** und entspricht somit demjenigen, das auch zum Zellaufschluss mittels Kombination von Sprühverfahren und Dekompression verwendet wird. Die Extraktion selbst wird nach Standardbedingungen des Stands der Technik durchgeführt. Die extrahierten Substanzen werden einschließlich des Lösungsmittels in Strom **13** zu einem Behälter **14** transportiert, der als Abscheider fungiert, in dem die extrahierten Substanzen nach Druckabsenkung vom Lösungsmittel getrennt werden. Das dabei zurück gewonnene Lösungsmittel wird über einer Rückführung **15** wieder in den Vorratsbehälter **4** zur Aufnahme des Lösungsmittels überführt und die extrahierten Substanzen werden dem Behälter **14** als Strom **18** entnommen.

Alternativ wird das Lösungsmittel **17** zur Extraktion in den Behälter **14** geleitet, der in diesem Fall als Extraktionsbehälter fungiert, indem das Lösungsmittel im Gegenstrom zu Strom **13,** der in diesem Fall das Zellaufschlussmaterial aus dem Behälter **12** darstellt, geführt wird. Dabei wird die Extraktion nach Standardbedingungen des Stands der Technik durchgeführt. Der Druck wird bei der Extraktion im Vergleich zu dem Druck, der im Behälter **12** herrscht, beibehalten. Bei dieser Verfahrensvariante ist dann ein zusätzlicher Behälter nötig, der als Abscheider fungiert (in Fig. 1 nicht gezeigt).

Die dabei einzuhaltenden Prozessparameter sind bei der Verwendung von CO₂ als Lösungsmittel **5** in einem Druckbereich von 300 bis 2500 bar, in einem Temperaturbereich von 10°C bis 90°C und in einem Verhältnis von Lösungsmittel 5 zu biogenem, suspendierten Ausgangsmaterial **2** von 5 bis 90 kg/kg optimal. Wird das Verfahren unter Verwendung von C2 - C4-Kohlenwasserstoffen durchgeführt, kann der Druckbereich auf Werte zwischen 100 bis 2500 bar festgelegt werden, wobei das Verhältnis von Lösungsmittel **5** zu biogenem, suspendiertem Ausgangsmaterial **2** auf 1 bis 60 kg/kg zu begrenzen ist.
Das Verfahren kann sowohl im Batch-Betrieb, als auch im kontinuierlichen Verfahren betrieben werden.

Durch die damit definierten Prozessbedingungen, die sich durch einen sehr hohen Druck, geringe Temperaturen und einer möglichen kontinuierlichen Fahrweise des Prozesses auszeichnen, können die gewünschten zellulären Wertstoffe in hoher Konzentration und in hoher Reinheit gewonnen werden.

Nachfolgend soll anhand zweier Beispiele das erfindungsgemäße Verfahren von dem aus dem Stand der Technik wie es beispielsweise in der WO91/01367A1 beschrieben wird, abgegrenzt werden.

### Beispiel 1:

A. Erfindungsgemäßes Verfahren:
   1 kg wässrige Suspension der Alge *Hematococcus pluvialis,* die eine Feststoffkonzentration von 181,1g/kg und einen Ölgehalt von ca. 13.7 wt% (bezogen auf das Trockengewicht) aufweist, wird mit einer Pumpe auf einen Druck von 1500 bar gebracht und anschließend mit dem Lösungsmittel CO₂ unter Druck gemischt. Das dabei entstandene Lösungsgemisch wird durch eine Düse in einen Druckbehälter mit einem niedrigeren Druck von 300 bar versprüht. Durch die kombinierte Dekompression mit dem Vorgang des Versprühens werden die Algen im Lösungsgemisch aufgeschlossen und das Lösungsgemisch im Druckbehälter fein versprüht. Das versprühte Lösungsgemisch mit den aufgeschlossenen Algen wird anschließend im Gegenstrom mit dem Lösungsmittel CO₂ im Druckbehälter ohne Druckänderung bei 300 bar extrahiert. Die extrahierten Substanzen werden mit dem Lösungsmittel CO₂ zu einem Abscheider transportiert, in dem die extrahierten Substanzen nach Druckabsenkung vom Lösungsmittel getrennt werden. In diesem Beispiel beträgt das Verhältnis des Lösungsmittel CO₂ zu biogenem, suspendierten Ausgangsmaterial 90 kg/kg.
      Das in diesem Fall erhaltene Produkt beträgt ca. 0,27 kg und besteht aus einer Emulsion aus Öl und Wasser, wobei die ölhaltige Phase durch Sedimentation abgetrennt wird. Daraus resultiert dann eine Gesamtmenge an gewonnenem Öl von 20,1 g/kg der Algensuspension.
B. Verfahren aus dem Stand der Technik:
   1 kg wässrige Algensuspension der Alge *Hematococcus pluvialis,* die eine Feststoffkonzentration von 181,1g/kg und einen Ölgehalt von ca. 13.7 wt% (bezogen auf das Trockengewicht) aufweist, wird mit Hilfe einer Pumpe auf einen Druck von 1500 bar gebracht und anschließend mit dem Lösungsmittel CO₂ unter Druck gemischt. Das dabei entstandene Lösungsgemisch wird durch eine Düse in einen Behälter auf Atmosphärendruck entspannt, aber nicht fein versprüht. Durch die Dekompression werden die Algen im Lösungsgemisch aufgeschlossen. Anschließend wird die aufgeschlossene Algensuspension in einen Druckbehälter mit einem Druck von 300 bar gefördert und dort versprüht. Das versprühte Lösungsgemisch mit den aufgeschlossenen Algen wird im Gegenstrom mit dem Lösungsgemisch extrahiert. Bei diesem Verfahren erfolgt die Dekompression und das Versprühen also in zwei aufeinanderfolgenden Prozessschritten und nicht simultan. Die extrahierten Substanzen werden anschließend mit dem Lösungsmittel zu einem Abscheider transportiert, in dem die extrahierten Substanzen nach Druckabsenkung vom Lösungsmittel getrennt werden. In diesem Beispiel beträgt das Verhältnis des Lösungsmittel CO₂ zu biogenem, suspendierten Ausgangsmaterial 110 kg/kg.
      Das in diesem Fall erhaltene Produkt beträgt ca. 0,20 kg und besteht aus einer Emulsion aus Öl und Wasser, wobei die ölhaltige Phase durch Sedimentation abgetrennt wird. Daraus resultiert dann eine Gesamtmenge an gewonnenem Öl von 15,8 g/kg der Algensuspension.

Die nach Beispiel 1 erhaltenen Ergebnisse können so interpretiert werden, dass eine Erhöhung der Produktausbeute von 21,4% durch das erfindungsgemäße Verfahren erzielt werden konnte.

### Beispiel 2:

A. Erfindungsgemäßes Verfahren:
   1 kg wässrige Suspension der Alge *Hematococcus pluvialis,* die eine Feststoffkonzentration von 181,1 g/kg und einen Ölgehalt von ca. 13.7 wt% (bezogen auf das Trockengewicht) aufweist, wird mit einer Pumpe auf einen Druck von 1000 bar gebracht und anschließend mit dem Lösungsmittel Propan unter Druck gemischt. Das dabei entstandene Lösungsgemisch wird durch eine Düse in einen Druckbehälter mit einem niedrigeren Druck von 50 bar versprüht. Durch die kombinierte Dekompression mit dem Vorgang des Versprühens werden die Algen im Lösungsgemisch aufgeschlossen und das Lösungsgemisch im Druckbehälter fein versprüht. Das versprühte Lösungsgemisch mit den aufgeschlossenen Algen wird anschließend im Gegenstrom mit dem Lösungsmittel Propan im Druckbehälter ohne Druckänderung bei 50 bar extrahiert. Die extrahierten Substanzen werden mit dem Lösungsmittel zu einem Abscheider transportiert, in dem die extrahierten Substanzen nach Druckabsenkung vom Lösungsmittel getrennt werden. In diesem Beispiel beträgt das Verhältnis des Lösungsmittel Propan zu biogenem, suspendierten Ausgangsmaterial 8 kg/kg.
      Das in diesem Fall erhaltene Produkt besteht aus nahezu reinem Öl. Die Gesamtmenge an gewonnenem Öl beträgt 22,1 g/kg der Algensuspension.
A. Verfahren aus dem Stand der Technik:
   1 kg wässrige Algensuspension der Alge *Hematococcus pluvialis,* die eine Feststoffkonzentration von 181,1g/kg und einen Ölgehalt von ca. 13.7 wt% (bezogen auf das Trockengewicht) aufweist, wird mit Hilfe einer Pumpe auf einen Druck von 1000 bar gebracht und anschließend mit dem Lösungsmittel Propan unter Druck gemischt. Das dabei entstandene Lösungsgemisch wird durch eine Düse in einen Behälter auf Atmosphärendruck entspannt, aber nicht fein versprüht. Durch die Dekompression werden die Algen im Lösungsgemisch aufgeschlossen. Anschließend wird die aufgeschlossene Algensuspension in einen Druckbehälter mit einem Druck von 50 bar gefördert und dort versprüht. Das versprühte Lösungsgemisch mit den aufgeschlossenen Algen wird im Gegenstrom mit dem Lösungsgemisch extrahiert. Bei diesem Verfahren erfolgt die Dekompression und das Versprühen also in zwei aufeinanderfolgenden Prozessschritten und nicht simultan. Die extrahierten Substanzen werden anschließend mit dem Lösungsmittel zu einem Abscheider transportiert, in dem die extrahierten Substanzen nach Druckabsenkung vom Lösungsmittel getrennt werden. In diesem Beispiel beträgt das Verhältnis des Lösungsmittels Propan zu biogenem, suspendierten Ausgangsmaterial 11 kg/kg.
      Das in diesem Fall erhaltene Produkt besteht aus nahezu reinem Öl. Die Gesamtmenge an gewonnenem Öl beträgt 18,8 g/kg der Algensuspension.

Die nach Beispiel 2 erhaltenen Ergebnisse können so interpretiert werden, dass eine Erhöhung der Produktausbeute von 15% durch das erfindungsgemäße Verfahren erzielt werden konnte.

### Beispiel 3:

A. Erfindungsgemäßes Verfahren:
   0,9 kg wässrige Suspension der Alge *Hematococcus pluvialis,* die eine Feststoffkonzentration von 181,1 g/kg und einen Astaxanthingehalt von ca. 3,0 wt% (bezogen auf das Trockengewicht) aufweist, wird mit einer Pumpe auf einen Druck von 2400 bar gebracht und anschließend mit dem Lösungsmittel CO₂ unter Druck gemischt. Das dabei entstandene Lösungsgemisch wird durch eine Düse in einen Druckbehälter mit einem niedrigeren Druck von 500 bar versprüht. Durch die kombinierte Dekompression mit dem Vorgang des Versprühens werden die Algen im Lösungsgemisch aufgeschlossen und das Lösungsgemisch im Druckbehälter fein versprüht. Das versprühte Lösungsgemisch mit den aufgeschlossenen Algen wird anschließend im Gegenstrom mit dem Lösungsmittel CO₂ im Druckbehälter ohne Druckänderung bei 500 bar extrahiert. Die extrahierten Substanzen werden mit dem Lösungsmittel CO₂ zu einem Abscheider transportiert, in dem die extrahierten Substanzen nach Druckabsenkung vom Lösungsmittel getrennt werden. In diesem Beispiel beträgt das Verhältnis des Lösungsmittels CO₂ zu biogenem, suspendierten Ausgangsmaterial 88 kg/kg.
      Das in diesem Fall erhaltene Produkt beträgt ca. 0,29 kg und besteht aus einer Emulsion aus Öl, Astaxanthin und Wasser, wobei die ölhaltige Phase durch Sedimentation abgetrennt wird. Aus der Öl-haltigen Phase kann dann Astaxanthin mit einer Ausbeute von 88,1% bezogen auf den Gesamtastaxanthingehalt des eingesetzten Algenmaterials, isoliert werden.
B. Verfahren aus dem Stand der Technik:
   0,9 kg wässrige Algensuspension der Alge *Hematococcus pluvialis,* die eine Feststoffkonzentration von 181.1 g/kg und einen Astaxanthingehalt von ca. 3,0 wt% (bezogen auf das Trockengewicht) aufweist, wird mit Hilfe einer Pumpe auf einen Druck von 2400 bar gebracht und anschließend mit dem Lösungsmittel CO₂ unter Druck gemischt. Das dabei entstandene Lösungsgemisch wird durch eine Düse in einen Behälter auf Atmosphärendruck entspannt, aber nicht fein versprüht. Durch die Dekompression werden die Algen im Lösungsgemisch aufgeschlossen. Anschließend wird die aufgeschlossene Algensuspension in einen Druckbehälter mit einem Druck von 500 bar gefördert und dort versprüht. Das versprühte Lösungsgemisch mit den aufgeschlossenen Algen wird im Gegenstrom mit dem Lösungsgemisch extrahiert. Bei diesem Verfahren erfolgt die Dekompression und das Versprühen also in zwei aufeinanderfolgenden Prozessschritten und nicht simultan. Die extrahierten Substanzen werden anschließend mit dem Lösungsmittel zu einem Abscheider transportiert, in dem die extrahierten Substanzen nach Druckabsenkung vom Lösungsmittel getrennt werden. In diesem Beispiel beträgt das Verhältnis des Lösungsmittel CO₂ zu biogenem, suspendierten Ausgangsmaterial 115 kg/kg.
      Das in diesem Fall erhaltene Produkt beträgt ca. 0,22 kg und besteht aus einer Emulsion aus Öl, Astaxanthin und Wasser, wobei die ölhaltige Phase durch Sedimentation abgetrennt wird. Aus der Öl-haltigen Phase kann dann Astaxanthin mit einer Ausbeute von 72,3% bezogen auf den Gesamtastaxanthingehalt des eingesetzten Algenmaterials, isoliert werden.

Die nach Beispiel 3 erhaltenen Ergebnisse können so interpretiert werden, dass eine Erhöhung der Produktausbeute von 21,8% durch das erfindungsgemäße Verfahren erzielt werden konnte.

### Beispiel 4:

A. Erfindungsgemäßes Verfahren:
   0,9 kg wässrige Suspension der Alge *Hematococcus pluvialis,* die eine Feststoffkonzentration von 181,1g/kg und einen Astaxanthingehalt von ca. 3,0 wt% (bezogen auf das Trockengewicht) aufweist, wird mit einer Pumpe auf einen Druck von 1600 bar gebracht und anschließend mit dem Lösungsmittel Butan unter Druck gemischt. Das dabei entstandene Lösungsgemisch wird durch eine Düse in einen Druckbehälter mit einem niedrigeren Druck von 150 bar versprüht. Durch die kombinierte Dekompression mit dem Vorgang des Versprühens werden die Algen im Lösungsgemisch aufgeschlossen und das Lösungsgemisch im Druckbehälter fein versprüht. Das versprühte Lösungsgemisch mit den aufgeschlossenen Algen wird anschließend im Gegenstrom mit dem Lösungsmittel Butan im Druckbehälter ohne Druckänderung bei 150 bar extrahiert. Die extrahierten Substanzen werden mit dem Lösungsmittel Butan zu einem Abscheider transportiert, in dem die extrahierten Substanzen nach Druckabsenkung vom Lösungsmittel getrennt werden. In diesem Beispiel beträgt das Verhältnis des Lösungsmittel Butan zu biogenem, suspendierten Ausgangsmaterial 8 kg/kg.
      Das in diesem Fall erhaltene Produkt beträgt ca. 26,1 g und besteht aus Öl und Astaxanthin. Aus der Öl-haltigen Phase kann dann Astaxanthin mit einer Ausbeute von 92,6% bezogen auf den Gesamtastaxanthingehalt des eingesetzten Algenmaterials, isoliert werden.
B. Verfahren aus dem Stand der Technik:
   0,9 kg wässrige Algensuspension der Alge *Hematococcus pluvialis,* die eine Feststoffkonzentration von 181,1g/kg und einen Astaxanthin gehalt von ca. 3,0 wt% (bezogen auf das Trockengewicht) aufweist, wird mit Hilfe einer Pumpe auf einen Druck von 1600 bar gebracht und anschließen mit dem Lösungsmittel Butan unter Druck gemischt. Das dabei entstandene Lösungsgemisch wird durch eine Düse in einen Behälter auf Atmosphärendruck entspannt, aber nicht fein versprüht. Durch die Dekompression werden die Algen im Lösungsgemisch aufgeschlossen. Anschließend wird die aufgeschlossene Algensuspension in einen Druckbehälter mit einem Druck von 150 bar gefördert und dort versprüht. Das versprühte Lösungsgemisch mit den aufgeschlossenen Algen wird im Gegenstrom mit dem Lösungsgemisch extrahiert. Bei diesem Verfahren erfolgt die Dekompression und das Versprühen also in zwei aufeinanderfolgenden Prozessschritten und nicht simultan. Die extrahierten Substanzen werden anschließend mit dem Lösungsmittel zu einem Abscheider transportiert, in dem die extrahierten Substanzen nach Druckabsenkung vom Lösungsmittel getrennt werden. In diesem Beispiel beträgt das Verhältnis des Lösungsmittel Butan zu biogenem, suspendierten Ausgangsmaterial 11 kg/kg.
      Das in diesem Fall erhaltene Produkt beträgt ca. 22,0 g und besteht aus Öl und Astaxanthin. Aus der Öl-haltigen Phase kann dann Astaxanthin mit einer Ausbeute von 78,4% bezogen auf den Gesamtastaxanthingehalt des eingesetzten Algenmaterials, isoliert werden.

Die nach Beispiel 4 erhaltenen Ergebnisse können so interpretiert werden, dass eine Erhöhung der Produktausbeute von 18,1% durch das erfindungsgemäße Verfahren erzielt werden konnte.

Vorteile, die sich aus der Erfindung ergeben:
- Es handelt sich um eine schonende Methode des Zellaufschlusses, da die chemische und physikalische Belastung, die auf die inneren Zellbestandteile wirkt, nur gering sind.
- Steigerung des Aufschlussgrades der Proben, bis hin zum Aufschluss schwer zugänglicher subzelluärer Organellen, wie Zellkerne oder Mitochondrien
- einheitlicher Homogenisierungsgrad nach dem Zellaufschluss erleichtert die nachfolgende Extraktion.
- auf eine aufwendige Trocknung des Ausgangsmaterials kann verzichtet werden.
- hohe Konzentration und hohe Reinheit der gewünschten zellulären Wertstoffe

### Bezugszeichenliste

- 1: Vorratsbehälter zur Aufnahme biogenem, suspendiertem Ausgangsmaterials
- 2: biogenes, suspendiertes Ausgangsmaterial
- 3: Pumpe
- 4: Vorratsbehälter
- 5: Lösungsmittel
- 6: Pumpe
- 7: unter Druck stehendes biogenes, suspendiertes Ausgangsmaterial
- 8: unter Druck stehendes Lösungsmittel
- 9: Lösungsgemisch
- 10: Druckmessgerät
- 11: Einspritzdüsen
- 12: Behälter
- 13: Strom
- 14: Behälter
- 15: Rückführung
- 16: Lösungsmittel zur Extraktion
- 17: Lösungsmittel
- 18: Strom
- 19: extrahierte Substanzen

## Patentansprüche

1. Verfahren zum Zellaufschluss von biogenen, suspendierten Ausgangsmaterialen mittels Kombination von Druckbeaufschlagung, Versprühen und Dekompression mit anschließender selektiver Extraktion und Abscheidung zellulärer Wertstoffe, wobei
• mindestens ein Vorratsbehälter als Vorlage für eine Suspension aus biogenem Ausgangsmaterial dient und
• mindestens ein weiterer Vorratsbehälter als Vorlage für ein Lösungsmittel verwendet wird,
• in einer Einheit zum Zellaufschluss ein Zellextrakt hergestellt wird, nachfolgend
• in einer Extraktionsstufe das Zellextrakt von Gas durchströmt wird und
• das mit zellulären Wertstoffen beladene Gas in einer Abscheidestufe unter Druckabsenkung von den zellulären Wertstoffen abgetrennt wird,
• die Suspension aus biogenem Ausgangsmaterial mittels einer Vorrichtung zur Druckerhöhung auf einen Druck von 100 - 2500 bar gebracht wird,
• das Lösungsmittel mittels einer Vorrichtung zur Druckerhöhung auf einen Druck von 100 - 2500 bar gebracht wird,
• das Lösungsmittel und die Suspension in einer Leitung unter einem Druck von 100 - 2500 bar zusammengeführt und zu einem Lösungsgemisch vermischt werden,
**dadurch gekennzeichnet, dass**
• das Lösungsgemisch über mindestens eine Düse unter einem Druck von 100 - 2500 bar und einer Temperatur von 10 - 90°C in einen Behälter, der einen geringeren Druck aufweist, versprüht wird.

2. Verfahren nach Anspruch 1, **dadurch gekennzeichnet, dass** das Lösungsmittel, mit dem die Suspension aus biogenem Ausgangsmaterial gemischt wird, aus einer Gruppe ausgewählt wird, die Ethan, Propan, Butan, Kohlenstoffdioxid, Lachgas, Ethylen, Propylen, Buthylen, weitere gesättigte oder ungesättigte Kohlenwasserstoffe, . Dimethylether, Schwefelhexaflourid Freone, wie zum Beispiel R 134a, R125, R32, R141b, und Mischungen daraus enthält.

3. Verfahren nach einem der Ansprüche 1 oder 2, **dadurch gekennzeichnet, dass** das Lösungsmittel, mit dem die Suspension aus biogenem Ausgangsmaterial gemischt wird, ein überkritisches Fluid ist, welches kein Kohlenwasserstoff ist.

4. Verfahren nach einem der Ansprüche 1 bis 3, **dadurch gekennzeichnet, dass** die Suspension aus biogenem Ausgangsmaterial vor dem Versprühen mit dem Lösungsmittel gesättigt wird.

5. Verfahren nach einem der Ansprüche 1 bis 3, **dadurch gekennzeichnet, dass** die Suspension aus biogenem Ausgangsmaterial vor dem Versprühen mit dem Lösungsmittel übersättigt wird.

6. Verfahren nach einem der Ansprüche 1 bis 5, **dadurch gekennzeichnet, dass** das verwendete Lösungsmittel zurückgewonnen und in den Vorratsbehälter für Lösungsmittel zurückgeführt wird.

7. Verfahren nach einem der Ansprüche 1 bis 6, **dadurch gekennzeichnet, dass** das biogene Ausgangsmaterial vor Bildung der Suspension durch Waschen, Filtrieren, Zerkleinern, Mahlen oder Sieben vorbehandelt wird.

8. Verfahren nach einem der Ansprüche 1 bis 7, **dadurch gekennzeichnet, dass** das Verfahren zum Zellaufschluss von biogenem, suspendiertem Ausgangsmaterial mittels Kombination von Druckbeaufschlagung, Versprühen und Dekompression mit anderen Aufschlussverfahren verknüpft wird, die aus einer Gruppe mechanischer Verfahren ausgewählt werden, die den Zellaufschluss mittels Hochdruckhomogenisator, Kugelmühle, Ultaschallhomogenisator, French Press und Prallstrahlapparaturen enthält.

9. Verfahren nach einem der Ansprüche 1 bis 8 **dadurch gekennzeichnet, dass** das Verfahren zum Zellaufschluss von biogenem, suspendiertem Ausgangsmaterial mittels Kombination von Druckbeaufschlagung, Versprühen und Dekompression mit anderen Aufschlussverfahren verknüpft wird, die aus einer Gruppe chemischer Methoden ausgewählt werden, die den Zellaufschluss mittels Antibiotika, Chelatbildner, Chaotrope Agenzien, Detergenzien und Alkalische Behandlung enthält.

10. Verfahren nach einem der Ansprüche 1 bis 9, **dadurch gekennzeichnet, dass** das Verfahren zum Zellaufschluss von biogenem, suspendiertem Ausgangsmaterial mittels Kombination von Druckbeaufschlagung, Versprühen und Dekompression mit anderen Aufschlussverfahren verknüpft wird, die aus einer Gruppe biologischer Methoden ausgewählt werden, die den Zellaufschluss mittels Enzymen, Phagen oder Autolyse enthält.

11. Verfahren nach einem der Ansprüche 1 bis 10, **dadurch gekennzeichnet, dass** das Verfahren zum Zellaufschluss von biogenem, suspendiertem Ausgangsmaterial mittels Kombination von Druckbeaufschlagung, Versprühen und Dekompression mit anderen Aufschlussverfahren verknüpft wird, die aus einer Gruppe physikalischer Methoden ausgewählt werden, die den Zellaufschluss mittels Einfrieren und Auftauen, Thermolyse oder Dekompression enthält.

12. Verfahren nach einem der Ansprüche 1 bis 11, **dadurch gekennzeichnet, dass** das verwendete biogene, suspendierte Ausgangsmaterial Algen sind.

13. Verfahren nach einem der Ansprüche 1 bis 12, **dadurch gekennzeichnet, dass** zelluläre Wertstoffe der Carotinoidklasse, wie Carotine oder Xanthophylle, die im biogenen, suspendierten Ausgangsmaterial enthalten sind, extrahiert werden.

14. Verfahren nach einem der Ansprüche 1 bis 13, **dadurch gekennzeichnet, dass** der zelluläre Wertstoff Astaxanthin, der im biogenen, suspendierten Ausgangsmaterial enthalten ist, extrahiert wird.

15. Verfahren nach einem der Ansprüche 1 bis 14, **dadurch gekennzeichnet, dass** zelluläre Wertstoffe der Stoffklasse der Fette und Öle, die im biogenen, suspendierten Ausgangsmaterial enthalten sind, extrahiert werden.

16. Verfahren nach einem der Ansprüche 1 bis 15, **dadurch gekennzeichnet, dass** das Verhältnis von Lösungsmittel zu biogenem, suspendiertem Ausgangsmaterial in einem Bereich von 1 bis 90 kg/kg liegt.

## Claims

1. Method for cell disruption of biogenic suspended raw materials by means of a combination of pressurization, atomization and decompression with a subsequent selective extraction and separation of cellular valuable substances, wherein
• at least one reservoir cubicle serves as reservoir for a suspension composed of biogenic raw material, and
• at least another reservoir cubicle is utilized as reservoir for a solvent,
• a cellular extract is produced in one unit for cell disruption, and subsequently
• the cellular extract is flown through by gas in an extraction stage, and
• the gas burdened with cellular valuable substances is separated from the cellular valuable substances in a separation stage by lowering the pressure,
• the suspension composed of biogenic raw material is pressurized to a pressure of 100 - 2500 bar by a device for pressure boosting,
• the solvent is pressurized to a pressure of 100 - 2500 bar by a device for pressure boosting,
• the solvent and the suspension are brought together in one line at a pressure of 100 - 2500 bar and mixed to a solution mixture,
**characterized in that**
• the solution mixture is atomized through at least one jet at a pressure of 100 to 2500 bar and a temperature of 10 to 90°C into a cubicle with a lower pressure.

2. Method according to claim 1, **characterized in that** the solvent with which the suspension of biogenic raw material is mixed is selected from a group that contains ethane, propane, butane, carbon dioxide, nitrous oxide, ethylene, propylene, butylene, other saturated or unsaturated hydrocarbons, dimethyl ether, sulphur hexafluoride, freons, e.g. R 134a, R125, R32, R141b, and mixtures thereof.

3. Method according to claim 1 or 2, **characterized in that** the solvent with which the suspension composed of biogenic raw material is mixed is a supercritical fluid that is not a hydrocarbon.

4. Method according to one of claims 1 to 3, **characterized in that** the suspension composed of biogenic raw material is saturated with the solvent prior to atomization.

5. Method according to one of claims 1 to 3, **characterized in that** the suspension composed of biogenic raw material is oversaturated with the solvent prior to atomization.

6. Method according to one of claims 1 to 5, **characterized in that** the applied solvent is recovered and returned into the reservoir cubicle for solvents.

7. Method according to one of claims 1 to 6, **characterized in that** the biogenic raw material is pretreated by scrubbing, filtering, crushing, grinding or screening prior to the formation of the suspension.

8. Method according to one of claims 1 to 7, **characterized in that** the method for cell disruption of biogenic suspended raw material by means of a combination of pressurization, atomization and decompression is linked to other disruption methods that are selected from a group of mechanical methods that contains cell disruption by means of a high-pressure homogenizer, ball mill, ultrasonic homogenizer, French press and impact blast apparatuses.

9. Method according to one of claims 1 to 8 **characterized in that** the method for cell disruption of biogenic suspended raw material by means of a combination of pressurization, atomization and decompression is linked to other disruption methods that are selected from a group of chemical methods that contains cell disruption by means of antibiotics, chelate forming agents, chaotropic agents, detergents, and alkaline treatment.

10. Method according to one of claims 1 to 9, **characterized in that** the method for cell disruption of biogenic suspended raw material by means of a combination of pressurization, atomization and decompression is linked to other disruption methods that are selected from a group of biological methods that contains cell disruption by means of enzymes, phages, or autolysis.

11. Method according to one of claims 1 to 10, **characterized in that** the method for cell disruption of biogenic suspended raw material by means of a combination of pressurization, atomization and decompression is linked to other disruption methods that are selected from a group of physical methods that contains cell disruption by means of freezing and thawing, thermolysis or decompression.

12. Method according to one of claims 1 to 11, **characterized in that** the applied biogenic suspended raw material is algae.

13. Method according to one of claims 1 to 12, **characterized in that** cellular valuable substances of the class of carotenoids, like carotenes or xanthophylls, contained in the biogenic suspended raw material are extracted.

14. Method according to one of claims 1 to 13, **characterized in that** the cellular valuable substance astaxanthin contained in the biogenic suspended raw material is extracted.

15. Method according to one of claims 1 to 14, **characterized in that** cellular valuable substances from the substance class of fats and oils contained in the biogenic suspended raw material are extracted.

16. Method according to one of claims 1 to 15, **characterized in that** the ratio of solvent to biogenic suspended raw material is between 1 and 90 kg/kg.

## Revendications

1. Procédé de fractionnement cellulaire de substances de base biogènes suspendues au moyen d'une combinaison de pressurisation, pulvérisation et décompression avec extraction sélective suivante et isolement de matériaux valorisables cellulaires,
• un récipient au moins servant de collecteur pour une suspension de substances de base biogènes et
• un autre récipient au moins étant utilisé comme collecteur pour un solvant,
• un extrait cellulaire étant fabriqué dans une unité pour le fractionnement cellulaire, ensuite
• l'extrait cellulaire étant traversé par du gaz dans une phase d'extraction et le gaz chargé de matériaux valorisables cellulaires étant séparé des matériaux valorisables cellulaires dans une phase d'isolement sous baisse de pression,
• la suspension de matériaux valorisables biogènes étant amenée à une pression de 100 à 2 500 bars au moyen d'un dispositif d'augmentation de pression,
• le solvant étant amené à une pression de 100 à 2 500 bars au moyen d'un dispositif d'augmentation de pression,
• le solvant et la suspension étant réunis dans une conduite à une pression de 100 à 2 500 bars et mélangés pour former un mélange de solution,
**caractérisé en ce que**
• le mélange de solution est vaporisé au moyen d'une buse à une pression de 100 à 2 500 bars et une température de 10 à 90 °C dans un récipient qui a une faible pression.

2. Procédé selon la revendication 1, **caractérisé en ce que** le solvant avec lequel est mélangée la suspension de substances de base biogènes est choisi dans un groupe qui contient de l'éthane, du propane, du butane, du dioxyde de carbone , de l'oxyde nitreux, de l'éthylène, du propylène, du butylène, d'autres hydrocarbures saturés ou non saturés, de l'éther méthylique, de l'hexafluorure de soufre, des fréones, tels que R134a, R125, R32, R141b ainsi que des mélanges de ces derniers.

3. Procédé selon une des revendications 1 ou 2, **caractérisé en ce que** le solvant avec lequel est mélangée la suspension de substances de base biogènes est une fluide surcritique, qui n'est pas un carbure.

4. Procédé selon une des revendications 1 à 3, **caractérisé en ce que** la suspension de substances de base biogènes est saturée avec le solvant avant la pulvérisation.

5. Procédé selon une des revendications 1 à 3, **caractérisé en ce que** la suspension de substances de base biogènes est sursaturée avec le solvant avant la pulvérisation.

6. Procédé selon une des revendications 1 à 5, **caractérisé en ce que** le solvant utilisé est récupéré et reconduit dans le récipient pour solvant.

7. Procédé selon une des revendications 1 à 6, **caractérisé en ce que** les substances de base biogènes sont prétraitées avant la formation de la suspension par lavage, filtration, concassage, broyage ou tamisage.

8. Procédé selon une des revendications 1 à 7, **caractérisé en ce que** le procédé de fractionnement cellulaire de substances de base biogènes suspendues au moyen d'une combinaison de pressurisation, pulvérisation et décompression est combiné à d'autres procédés de fractionnement choisis dans un groupe de procédés mécaniques comprenant le fractionnement cellulaire au moyen d'homogénéisateur haute pression, broyeur à boulets, homogénéisateur ultrason, french press et appareils de choc.

9. Procédé selon une des revendications 1 à 8, **caractérisé en ce que** le procédé de fractionnement cellulaire de substances de base biogènes suspendues au moyen d'une combinaison de pressurisation, pulvérisation et décompression est combiné à d'autres procédés de fractionnement choisis dans un groupe de méthodes chimiques comprenant le fractionnement cellulaire au moyen d'antibiotiques, d'agents chélateurs, d'agents chaotropiques, de détergents et de traitement alcalin.

10. Procédé selon une des revendications 1 à 9, **caractérisé en ce que** le procédé de fractionnement cellulaire de substances de base biogènes suspendues au moyen d'une combinaison de pressurisation, pulvérisation et décompression est combiné à d'autres procédés de fractionnement choisis dans un groupe de méthodes biologiques contenant le fractionnement cellulaire au moyen d'enzymes, de phages ou d'autolyse.

11. Procédé selon une des revendications 1 à 10, **caractérisé en ce que** le procédé de fractionnement cellulaire de substances de base biogènes suspendues au moyen d'une combinaison de pressurisation, pulvérisation et décompression est combiné à d'autres procédés de fractionnement choisis dans un groupe de méthodes physiques comprenant le fractionnement cellulaire par congélation et décongélation, thermolyse ou décompression.

12. Procédé selon une des revendications 1 à 11, **caractérisé en ce que** les substances de base biogènes suspendues utilisées sont des algues.

13. Procédé selon une des revendications 1 à 12, **caractérisé en ce que** des matériaux valorisables cellulaires de la classe caroténoïde, tels que la carotène ou la xanthophylle, contenus dan les substances biogènes suspendues, sont extraits.

14. Procédé selon une des revendications 1 à 13, **caractérisé en ce que** le matériau valorisable astaxanthine, contenu dan les substances biogènes suspendues, est extrait.

15. Procédé selon une des revendications 1 à 14, **caractérisé en ce que** des matériaux valorisables cellulaires de la classe de substances des graisses et huiles, contenus dans les substances biogènes suspendues, sont extraits.

16. Procédé selon une des revendications 1 à 15, **caractérisé en ce que** le rapport du solvant par rapport aux substances de base biogènes suspendues se situe dans une plage de 1 à 90 kg/kg.
